Europäisches Patentamt

European Patent Office

Office européen des brevets

(11) Numéro de publication: **0 286 490**
**A1**

(12) **DEMANDE DE BREVET EUROPEEN**

(21) Numéro de dépôt: 88400677.6

(22) Date de dépôt: 21.03.88

(51) Int. Cl.⁴: **A 61 B 3/10**

(30) Priorité: 20.03.87 FR 8703906

(43) Date de publication de la demande:
12.10.88 Bulletin 88/41

(84) Etats contractants désignés: **ES GR**

(71) Demandeur: **INSTITUT NATIONAL DE LA SANTE ET DE LA RECHERCHE MEDICALE (INSERM)**
**101, rue de Tolbiac**
**F-75654 Paris Cédex 13 (FR)**

**DE SCAN**
**10 Rue de Presles**
**F-75015 Paris (FR)**

(72) Inventeur: **Gilbert, Jean Claude**
**34 Chaussée de l'Etang**
**F-94160 Saint Mandé (FR)**

(74) Mandataire: **Joly, Jean-Jacques et al**
**CABINET BEAU DE LOMENIE 55, rue d'Amsterdam**
**F-75008 Paris (FR)**

(54) Dispositif d'observation de l'oeil utilisant la réflexion d'un rayonnement Infrarouge par le globe oculaire.

(57) Le dispositif d'observation comprend une source (16) d'illumination de l'oeil par un rayonnement infrarouge, un filtre interférentiel (14) de séparation du rayonnement infrarouge réfléchi et de la lumière visible, un détecteur (10) fournissant une image comportant le point de réflexion et des moyens de traitement (22, 24, 20, 26) du signal analogique fourni par le détecteur. Le filtre interférentiel (14) est d'un type laissant passer le rayonnement infrarouge et réfléchissant la lumière visible. Le filtre (14) est placé à proximité immédiate du détecteur et est aligné avec le détecteur et avec l'oeil pour recevoir directement le rayonnement réfléchi par l'oeil, tandis que la source infrarouge (16) est placée à proximité immédiate de l'axe optique du détecteur pour illuminer directement l'oeil. L'image fournie par le détecteur comprend l'image de la pupille et celle du reflet de la source sur la cornée de sorte que le dispositif est utilisable en pupillométrie et pour la mesure des mouvements oculaires.

FIG.1

EP 0 286 490 A1

## Description

**Dispositif d'observation de l'oeil utilisant la réflexion d'un rayonnement infrarouge par le globe oculaire.**

La présente invention concerne un dispositif d'observation de l'oeil du type utilisant la réflexion d'un rayonnement infrarouge par le globe oculaire.

Des dispositifs comprenant une source d'illumination de l'oeil par un rayonnement infrarouge et un détecteur destiné à recevoir le rayonnement infrarouge rélfléchi sont utilisés dans des appareils de mesure visant à l'établissement d'un diagnostic en ophthalmologie ou destinés à des applications industrielles utilisant le suivi du regard. Deux raisons principales conduisent au choix d'un rayonnement infrarouge. La rétine est insensible au rayonnement infrarouge. Et, ce rayonnement étant moins énergétique que la lumière visible, les reflets parasites de l'éclairage infrarouge sur la surface oculaire très réfléchissante sont négligeables par rapport à ceux observés avec un éclairage en lumière visible.

A titre d'exemples d'application médicale, on peut citer, à titre non limitatif, la mesure des mouvements oculaires et la mesure des dimensions de la pupille pour l'évaluation du réflexe pupillaire. A titre d'exemples d'applications industrielles, on peut citer des systèmes automatiques de manipulation ou de guidage de poursuite d'un mobile, basés sur le suivi du regard et des systèmes de reconnaissance de formes situées à la surface du globe oculaire ou dans le globe oculaire lui-même.

Beaucoup de ces applications exigent que l'oeil puisse recevoir la lumière visible éclairant un champ de vision ou provenant d'une source. On connaît depuis très longtemps des dispositifs répondant à cette condition et dans lesquels, pour obtenir une image de l'oeil sur laquelle apparaît la zone de réflexion infrarouge, le détecteur est soustrait à l'action de la lumière visible par un filtre. L'article intitulé "Infrared video pupillometry : a method used to measure the pupillary effects of drugs in small laboratory animals in real time", par Rodney B. Murray et autre, dans Journal of Neuroscience Methods, 3 (1981) 365-375, montre un dispositif dans lequel le faisceau provenant de la source infrarouge est renvoyé vers l'oeil par une lame semi-réflechissante et l'image de l'oeil est formée sur une caméra de télévision à travers la lame semi-réfléchissante et un filtre infrarouge. Ce mode de séparation du spectre visible et du spectre infrarouge conduit à une absorption considérable de l'infrarouge, déjà peu énergétique. En conséquence, le dispositif doit être placé sur l'axe optique très près du globe oculaire, ce qui limite beaucoup le champ visuel. Il est difficilement exploitable lorsque l'oeil est mobile.

Dans un autre dispositif, décrit dans la demande de brevet DE- 31 24 192, le rayonnement infrarouge provenant de la source est renvoyé vers l'oeil par un miroir qui réfléchit totalement dans le domaine infrarouge mais est semi-réfléchissant dans le domaine visible. Le rayonnement réfléchi par l'oeil est renvoyé vers un détecteur par le même miroir et passe à travers un filtre interférentiel qui ne réfléchit que la lumière visible et qui est situé entre le miroir et le détecteur. Dans le but de mesurer les mouvements oculaires, le dispositif est prépositionné en utilisant une source de lumière visible située sur l'axe optique du filtre entre celui-ci et le miroir dont le faisceau est dirigé vers l'oeil après réflexion totale sur le filtre et réflexion sur le miroir semi-réfléchissant dans le domaine visible. La lumière visible réfléchie par l'oeil passe à travers le miroir semi-réfléchissant dans le domaine visible pour être reçue par un deuxième détecteur ou par l'oeil de l'expérimentateur. Le réglage de position est effectué pour conférer au rayonnement de lumière visible une direction prédéterminée après réflexion par l'oeil. Dans ce dispositif connu, la longueur du trajet optique parcouru par le rayonnement infrarouge de l'oeil au détecteur entraîne un affaiblissement important. De plus, le miroir réfléchit indistinctement le spectre en dehors de la plage émise, ce qui affecte le signal reçu par le deuxième détecteur d'un bruit de fond important.

L'invention a pour but de fournir un dispositif répondant mieux que ceux antérieurement connus aux exigences de la pratique, notamment en ce qu'il réduit la distorsion et l'affaiblissement du faisceau infrarouge atteignant le détecteur à une valeur pratiquement minimale, et assure un filtrage réduisant le bruit de fond auquel est soumis le détecteur tout en laissant subsister un large champ de vision dans le spectre visible.

L'invention a aussi pour but de fournir un dispositif qui autorise l'éclairement simultané du globe oculaire par une ou plusieurs sources visibles ponctuelles en même temps que par la source infrarouge et est utilisable tant pour des applications demandant la mesure de mouvements oculaires que pour des applications demandant la mesure d'une ou plusieurs dimensions de la pupille.

Dans ces buts, l'invention propose un dispositif comprenant :

- une source de rayonnement infrarouge disposée de manière à illuminer directement un oeil à observer,

- un détecteur destiné à recevoir le rayonnement infrarouge réfléchi par l'oeil à observer pour fournir une image comprenant au moins l'image de la pupille de l'oeil à observer,

- un filtre interférentiel placé devant le détecteur, à proximité immédiate de celui-ci, le filtre laissant passer le rayonnement infrarouge et réfléchissant la lumière visible, et le filtre et le détecteur étant disposés pour être alignés avec l'oeil à observer de manière à recevoir directement le rayonnement réfléchi par celui-ci, et

- des moyens de traitement reliés au détecteur et destinés à traiter l'image fournie par celui-ci afin de fournir au moins une information relative à la pupille de l'oeil à observer.

Ainsi, dans le dispositif selon l'invention, le rayonnement infrarouge produit par la source est dirigé directement vers l'oeil, c'est-à-dire sans optique de renvoi, et le rayonnement infrarouge réfléchi par l'oeil est dirigé directement vers le détecteur, à travers le filtre interférentiel, l'oeil, le filtre et le détecteur étant alignés sur un même axe optique.

Le détecteur utilisé fournit une image qui comprend au moins une image de la pupille; L'analyse de l'image fournie par le détecteur, éventuellement après numérisation, permet la reconnaissance de la pupille et la mesure de dimensions de celle-ci, par exemple le plus grand diamètre apparent et la surface. Le dispositif selon l'invention est donc bien adapté à une utilisation en pupillométrie. La disposition d'une ou plusieurs sources de lumière visible à proximité de l'axe optique permet d'engendrer des stimuli constitués par des éclats de lumière visible et d'utiliser alors le dispositif pour l'examen des réflexes pupillaires. Dans la mesure où le détecteur a un domaine angulaire d'observation suffisant, l'image qu'il fournit comprend non seulement l'image de la pupille mais également celle du reflet de la source infrarouge sur la cornée quelle que soit la position du globe oculaire. L'image du reflet fournit une information sur la position relative de la tête par rapport à la source tandis que l'image de la pupille fournit une information sur la position relative de la pupille, donc du globe oculaire par rapport à la source. Il est donc possible d'dn déduire la position du globe oculaire par rapport à la tête et donc de suivre les mouvements du globe oculaire, c'est-à-dire l'orientation du regard, avec un seuil détecteur.

Le détecteur utilisé est de type matriciel. On pourra notamment utiliser une caméra à matrice de capteurs CCD ou CTD (dispositifs à couplage ou transfert de charge).

Le filtre interférentiel transmet le rayonnement infrarouge en réfléchissant la lumière visible. Sa longueur d'onde de coupure basse doit se situer à la frontière des spectres visible et infrarouge, soit à environ 750 nm. La transparence maximale du filtre doit correspondre à la plage de longueurs l'onde de sensibilité maximale du détecteur.

Dans le cas de capteurs CCD ou CTD, la plage de sensibilité maximale va généralement d'environ 800 nm 0 1100 nm.

Du fait que le filtre interférentiel laisse sourtout passer le rayonnement qui provient de la source infrarouge, il assure, en plus de sa fonction de séparation des spectres, une fonction de filtrage optique réduisant le bruit de fond auquel est soumis le signal, ce qui facilite l'exploitation de ce dernier. L'absorption par le filtre est relativement faible dans la zone de sensibilité. L'incidence du faisceau provenant de l'oeil sur le filtre interférentiel peut varier autour de la normale sans pour autant provoquer une modification inacceptable de la transmission. Le filtre interférentiel ne gêne pas le champ visuel de l'oeil car il peut être aussi éloigné de l'oeil que le permettent la puissance du faisceau réfléchi par l'oeil et la sensibilité et la nature du détecteur.

L'invention sera mieux comprise à la lecture de la description qui suit d'un mode particulier de réalisation donné à titre d'exemple non limitatif. La description se réfère aux dessins qui l'accompagnent, dans lesquels :

- la figure 1 est un schéma de principe en perspective montrant une disposition relative possible des différents composants,

- la figure 2 montre la courbe représentative de la transparence du filtre interférentiel du dispositif de la figure 1, en fonction de la longueur d'onde.

Le dispositif qui sera maintenant décrit utilise la technique dite à "pupille noire".

Ce dispositif comprend une source de rayonnement infrarouge 16 destinée à illuminer l'oeil à observer 12 et un détecteur 10 destiné à recueillir le rayonnement infrarouge réfléchi par l'oeil, après passage à travers un filtre interférentiel 14 placé devant le détecteur 10, à proximité immédiate de celui-ci. Le détecteur 10 et le filtre 14 sont alignés sur un axe optique passant par le centre de rotation de l'oeil 12.

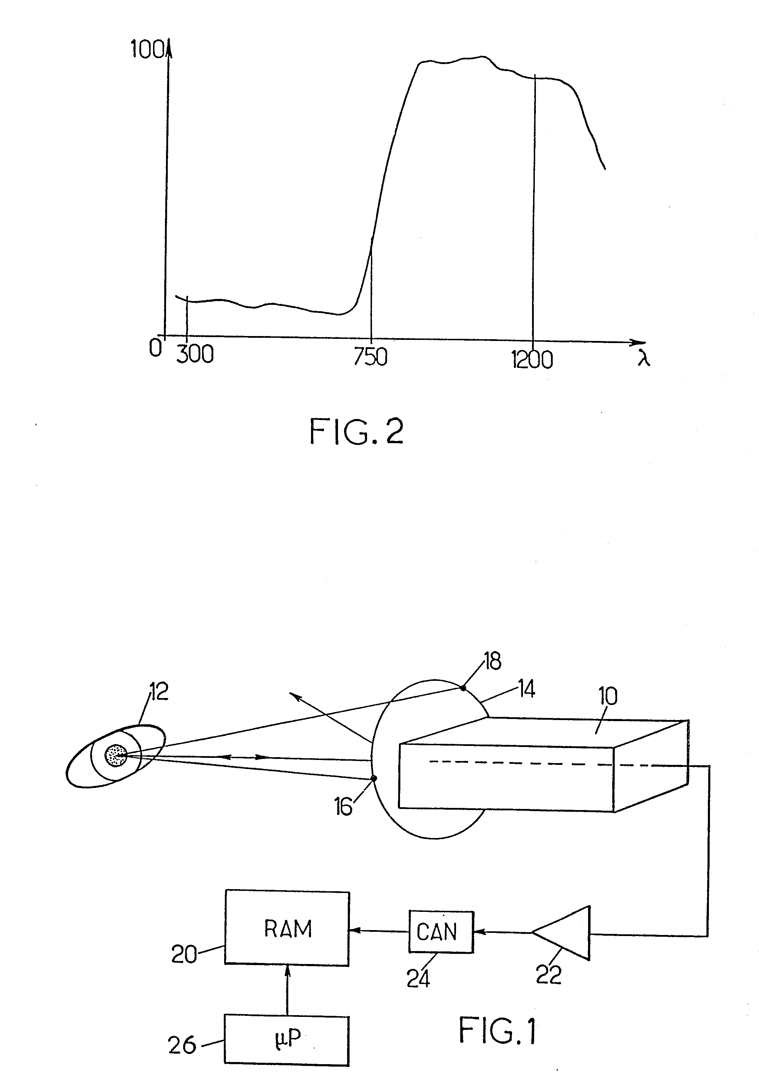

Le filtre interférentiel 14 soustrait le détecteur. On peut notamment utiliser un filtre interférentiel dont la transmission est représentée par une courbe du genre montré en figure 2, où $\underline{c}$ désigne le coefficient de transmission, $\lambda$ la longueur d'onde. Le support du filtre sera généralement en germanium, qui est transparent à l'infrarouge dans le domaine spectral à transmettre. Le nombre et l'épaisseur des couches interférentielles seront choisis de façon que l'affaiblissement subi par le faisceau provenant de l'oeil soit faible dans la plage de longueurs d'onde comprise entre 750 et 1300 nm environ. Par contre, le filtre 14 réfléchit la lumière de longueur d'onde inférieure, au-dessous de 630 nm au moins et soustrait donc la matrice de capteurs à l'effet de la lumière visible.

La source infrarouge 16 peut être une diode électroluminescente ou une diode laser, de longueur d'onde adaptée au filtre et au détecteur. Elle est placée à proximité de l'axe.

Lorsqu'on cherche à déterminer la réponse de l'oeil à des stimuli constitués par des éclats de lumière visible, au moins une source 18 de lumière visible peut être placée également à proximité immédiate de l'axe optique. La lumière visible réfléchie par le globe oculaire ne pourra atteindre le détecteur 10, étant renvoyée par le filtre interférentiel 14.

Plusieurs sources infrarouges 16, placées dans une position relative prédéterminée, peuvent être prévues au lieu d'une seule pour permettre une discrimination par rapport à des sources parasites éventuelles.

L'image analogique fournie par le détecteur 10 est stockée sous forme numérique dans une mémoire vive 20 constituant tampon. A cet effet, les signaux d'image analogique produits par le détecteur sont amplifiés au moyen d'un amplificateur 22 puis convertis sous forme numérique au moyen d'un convertisseur analogique-numérique 24 dont la sortie est reliée à la mémoire 20. Le traitement des informations stockées dans la mémoire 20, est effectué au moyen d'un circuit à microprocesseur 26 qui commande aussi (par des liaisons non représentées) l'extraction et la conversion des singaux analogiques fournis par le détecteur 10 et l'écriture dans la mémoire 20.

L'image fournie par le détecteur 10 comprend l'image de la pupille. En pupillométrie, les informations recherchées sont notamment le plus grand diamètre apparent et la surface de la pupille. Dans le cas envisagé d'un examen à pupille noire, l'image de la pupille se présente sous forme d'une tache avec un certain contraste par rapport aux autres zones de l'oeil. La reconnaissance de l'image de la pupille est réalisée en recherchant les éléments d'image (pixels) ayant une valeur de luminance particulière ou, de préférence, par reconnaissance de forme en identifiant le contour de l'image de la pupille. Il s'agit de procédés classiques de traitement d'image dont la mise en oeuvre est effectuée au moyen du circuit à microprocesseur 26 programmé à cet effet. Lorsque l'image de la pupille a été reconnue, la mesure de son plus grand diamètre apparent et la mesure et de sa surface peuvent être effectuées par calcul au moyen du circuit à microprocesseur 26. Ces mesures, en combinaison avec la commande de stimuli sous forme d'éclats de lumière visible produits par la source 18, permettent une détermination des réflexes pupillaires par calcul des variations des dimensions mesurées.

L'image fournie par le détecteur 10 comprend également le reflet de la source 16 sur la cornée. Ce reflet se présente sous forme d'une tache très brillante avec un fort contraste par rapport aux autres zones de l'oeil. Il suffit alors de rechercher les éléments d'image ayant la brillance maximale, c'est-à-dire ceux qui, en mémoire, sont codés avec la valeur numérique maximale. Si le reflet occupe plusieurs éléments d'image, le barycentre peut en être recherché au moyen d'un programme mis en oeuvre par le circuit à mircroprocesseur 26, par exemple selon la méthode décrite dans la demande de brevet français N° 2 382 056. On dispose ainsi d'une information de position du reflet de la source sur le globe oculaire.

Comme déjà indiqué, la connaissance de la position de la pupille et celle de la position du reflet de la source permettent d'effectuer un suivi du regard, c'est-à-dire une mesure des mouvements oculaires, en mettant en oeuvre des moyens optiques très simples.

Pour effectuer les traitements ci-dessus, il suffit d'effectuer une quantification de l'image fournie par le détecteur 10 sur un nombre de niveaux relativement faible, par exemple une quantification sur quatre niveaux (deux bits) au moyen du convertisseur 24.

Le dispositif, du fait de sa simplicité, se prête parfaitement à un examen binoculaire, que la complexité des systèmes antérieurs rendait difficile.

## Revendications

1. Dispositif d'observation de l'oeil utilisant la réflexion d'un rayonnement infrarouge par le globe oculaire, comprenant :
   - une source de rayonnement infrarouge disposée de manière à illuminer directement un oeil à observer,
   - un détecteur destiné à recevoir le rayonnement infrarouge réfléchi par l'oeil à observer pour fournir une image comprenant au moins l'image de la pupille de l'oeil à observer,
   - un filtre interférentiel placé devant le détecteur, à proximité immédiate de celui-ci, le filtre laissant passer le rayonnement infrarouge et réfléchissant la lumière visible, et le filtre et le détecteur étant disposés pour être alignés avec l'oeil à observer de manière à recevoir directement le rayonnement réfléchi par celui-ci, et
   - des moyens de traitement reliés au détecteur et destinés à traiter l'image fournie par celui-ci afin de fournir au moins une information relative à la pupille de l'oeil à observer.

2. Dispositif selon la revendication 1, caractérisé en ce que le détecteur est de type matriciel.

3. Dispositif selon la revendication 2, caractérisé en ce que le détecteur délivre un signal d'image analogique qui est converti sous forme numérique au moyen d'un convertisseur à faible nombre de niveaux de quantification.

4. Dispositif selon la revendication 3, caractérisé en ce que le conversion sous forme numérique s'effectue sous quatre niveaux.

5. Dispositif selon la revendication 1, caractérisé en ce que les moyens de traitement sont agencés pour fournir une information de position du reflet de la source infrarouge sur le globe oculaire de l'oeil à observer.

6. Dispositif selon la revendication 1, caractérisé en ce que les moyens de traitement sont agencés pour fournir une information de position de la pupille de l'oeil à observer, ainsi qu'une information de position du reflet de la source infrarouge sur le globe oculaire de l'oeil à observer, à partir de l'image fourni par le détecteur, de sorte qu'un suivi des mouvements du globe oculaire peut être effectué en utilisant un seul détecteur.

7. Dispositif selon la revendication 1, caractérisé en ce que les moyens de traitement sont agencés pour fournir au moins une information de dimension de la pupille de l'oeil à observer.

8. Dispositif seloln la revendication 6, caractérisé en ce qu'il comprend en outre au moins une source de lumière visible pour engendrer des stimuli sous forme d'éclats de lumière visible dirigés vers l'oeil à observer, et les moyens de traitement sont agencés pour fournir au moins une information de variation de dimension de la pupille de l'oeil à observer en réponse aux stimuli.

9. Dispositif selon la revendication 1, caractérisé en ce que le filtre comporte un support en germanium et des couches interférentielles telles qu'il présente une bande passante allant de 750 nm à au moins 1200 nm.

10. Dispositif selon la revendication 1, caractérisé en ce que la source (16) est constituée par une diode électroluminescente ou une diode laser.

0286490

FIG.2

FIG.1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,Y | FR-A-2 587 191 (INSERM et THOMSON CSF)<br>* Abrégé; page 3, lignes 13-18,25-33; page 4, lignes 6-12; page 7, lignes 11-33; page 8, lignes 5-22; page 10, ligne 13 - page 11, ligne 19; figures 1-5 * | 1,3,5-7,10 | A 61 B 3/10 |
| P,A | | 2 | |
| Y | WO-A-8 603 113 (P. UDDEN et al.)<br>* Abrégé; page 1, ligne 29 - page 2, ligne 11; page 2, ligne 24 - page 3, ligne 35; figures 1-3 * | 1,3,5-7,10 | |
| A | WO-A-8 604 799 (FAIRVILLE MEDICAL OPTICS, INC.)<br>* Abrégé; page 3, lignes 7-22; page 5, lignes 1-24; page 9, ligne 3 - page 10, ligne 18; figures 1-9 * | 1,5-8,10 | |
| A | ISA TRANSACTIONS, vol. 19, no. 4, 1980, pages 3-6, ISA; G.A. RINARD et al.: "An infrared system for determining ocular position"<br>* Page 3, l'abrégé; pages 4,5, les paragraphes: "Present eye position encoder" et "TTL scanning method"; figure 2 * | 1,2,5,6,10 | **DOMAINES TECHNIQUES RECHERCHES (Int. Cl.4)**<br><br>A 61 B |
| A | DE-C-3 124 192 (D. BOUIS)<br>* Colonne 5, ligne 8 - colonne 6, ligne 67; figures 1a-2 * | 1,5,6,10 | |
| A | GB-A-1 578 136 (HAWKER SIDDELEY AVIATION LTD)<br>* Page 2, lignes 26-63; figure 2 * | 1,2,5,6,10 | |

-/-

Le présent rapport a été établi pour toutes les revendications

| Lieu de la recherche | Date d'achèvement de la recherche | Examinateur |
|---|---|---|
| LA HAYE | 24-06-1988 | RIEB K.D. |

## DOCUMENTS CONSIDÉRES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | JOURNAL OF NEUROSCIENCE METHODS, vol. 3, 1981, pages 365-375, Elsevier North Holland Biomedical Press, Amsterdam, NL; R.B. MURRAY et al.: "Infrared video pupillometry: a method used to measure the pupillary effects of drugs in small laboratory animals in real time" * En entier * | 1,2,5,6 ,10 | |
| D,A | FR-A-2 382 056 (INSERM) * Page 15, lignes 7-17; page 16, ligne 30 - page 17, ligne 35; figures 1,4 * | 1,3,5-7 ,10 | |

DOMAINES TECHNIQUES
RECHERCHES (Int. Cl.4)

Le présent rapport a été établi pour toutes les revendications

| Lieu de la recherche | Date d'achèvement de la recherche | Examinateur |
|---|---|---|
| LA HAYE | 24-06-1988 | RIEB K.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)